# FASCICULE DE BREVET EUROPEEN

(11) **EP 3 302 643 B1**
(45) Date de publication et mention de la délivrance du brevet: **17.02.2021**
(21) Numéro de dépôt: 16736900.8
(22) Date de dépôt: 02.06.2016
(51) Int. Cl.: A61M 5/20, A61M 5/315, A61M 5/32

(54) **AUTOINJECTEUR AVEC UN SYSTEME RETARDATEUR DU DISPOSITIF D'INDICATION DU RETRAIT DE L'AUTOINJECTEUR**
AUTOINJEKTOR MIT EINEM SYSTEM ZUR VERZÖGERUNG DER VORRICHTUNG FÜR DIE ANZEIGE DER ENTFERNUNG DES AUTOINJEKTORS
AUTO-INJECTOR WITH A SYSTEM FOR DELAYING THE DEVICE INDICATING THE REMOVAL OF THE AUTO-INJECTOR

(30) Priorité: 05.06.2015 FR 1555160
(43) Date de publication de la demande: 11.04.2018
(73) Titulaire: Aptar France SAS, 27110 Le Neubourg (FR)
(72) Inventeur: FABIEN, David, 29810 Plouarzel (FR); GOMEZ, Thomas, 33160 Saint Aubin De Medoc (FR); HIS, Olivier, 27430 Saint Etienne Du Vauvray (FR); SAUSSAYE, Anthony, 61300 Saint Sulpice Sur Risle (FR)
(74) Mandataire: CAPRI
(86) Numéro de dépôt international: PCT/FR2016/051309
(87) Numéro de publication internationale: WO 2016/193620

(56) Documents cités:
- WO-A1-2013/078200
- WO-A1-2014/159017
- WO-A2-2008/112472
- WO-A2-2011/109205

## Description

La présente invention concerne un autoinjecteur.

Les autoinjecteurs sont bien connus dans l'état de la technique. Ces dispositifs ont principalement pour but de réaliser une injection automatique du contenu d'une seringue à l'intérieur du corps d'un patient. Divers systèmes existent pour rendre automatique la pénétration de l'aiguille dans le corps du patient ainsi que l'injection du produit fluide contenu dans la seringue. Les autoinjecteurs sont des dispositifs relativement complexes qui doivent répondre à un certain nombre d'exigences de contraintes pour être fiables. La robustesse du dispositif, sa maniabilité, et sa facilité d'utilisation pour l'utilisateur sont également des éléments importants. Par ailleurs, la plupart de ces autoinjecteurs étant à usage unique, le coût de fabrication et d'assemblage est également un facteur dont il faut tenir compte.

Il existe de nombreux autoinjecteurs sur le marché, qui présentent toutefois un certain nombre d'inconvénients.

Ainsi, notamment lorsque le volume de produit fluide est relativement important et/ou lorsque le produit fluide injecté est relativement visqueux, il est souhaitable de permettre au produit de diffuser du site d'injection pendant quelques secondes après ladite injection. Si l'utilisateur retire l'autoinjecteur immédiatement après la fin de l'injection, une partie du produit peut ressortir du corps de l'utilisateur ce qui diminue l'efficacité du traitement. Il est donc souhaitable de prévoir que l'utilisateur maintienne l'autoinjecteur contre son corps encore pendant quelques secondes après la fin de l'injection. Cet aspect est généralement résolu par les autoinjecteurs existants par la notice d'utilisation qui demande à l'utilisateur de compter dans sa tête un certain nombre de secondes avant de retirer le dispositif. Ceci n'est pas fiable et donc insatisfaisant, car le système dépend alors de l'utilisateur lui-même, qui dans certains cas peut être perturbé ou affaibli par l'action d'injection qu'il vient de réaliser.

Les documents WO2011109205, WO2014159017, WO2008112472 et WO2013078200 décrivent des autoinjecteurs de l'état de la technique.

La présente invention a pour but de fournir un autoinjecteur qui ne reproduit pas les inconvénients susmentionnés, et qui permet de répondre aux différentes exigences et contraintes importantes pour une utilisation sûre et fiable de l'autoinjecteur.

La présente invention a aussi pour but de fournir un autoinjecteur qui soit fiable d'utilisation, qui permet à l'utilisateur de déterminer quand il doit retirer ou quand il peut retirer l'autoinjecteur de son corps après son utilisation, qui soit sûr et qui empêche tout risque de blessure, et qui soit simple et peu coûteux à fabriquer et à assembler.

La présente invention a donc pour objet un autoinjecteur tel que décrit dans la revendication 1. Des modes de réalisation avantageux sont décrits dans les revendications dépendantes.

Ces caractéristiques et avantages et d'autres de la présente invention apparaîtront plus clairement au cours de la description détaillée suivante, faite en référence aux dessins joints, donnés à titre d'exemples non limitatifs, et sur lesquels :
Les figures 1a et 1b sont des vues schématiques, respectivement de côté et en section transversale, d'un autoinjecteur selon un premier mode de réalisation avantageux de la présente invention, en position de repos avant piquage,
Les figures 2a et 2b sont des vues similaires à celles des figures 1a et 1b, en position après piquage et avant injection,
Les figures 3a et 3b sont des vues similaires à celles des figures 2a et 2b, en position juste avant la fin d'injection et au moment du déclenchement du système retardateur,
Les figures 4a et 4b sont des vues similaires à celles des figures 3a et 3b, en début d'actionnement du système retardateur,
Les figures 5a à 5c sont des vues similaires à celles de la figure 4a, montrant trois positions d'indication entre le début et la fin d'actionnement du système retardateur,
La figure 5d est une vue similaire à celle de la figure 4b en fin d'actionnement du dispositif d'indication et avant retrait de l'autoinjecteur du site d'injection,
La figure 6 est une vue similaire à celle de la figure 5c, en position de fin d'utilisation, après retrait de l'autoinjecteur du site d'injection,
La figure 7 est une vue en perspective éclatée du système retardateur selon ledit premier mode de réalisation des figures 1 à 6,
Les figures 8 et 9 sont des vues en perspective de détail du système de la figure 7, montrant le réservoir de fluide formant l'élément indicateur du dispositif d'indication visuel, sonore et/ou tactile, respectivement vue d'en bas et d'en haut,
La figure 10 est une vue en perspective de détail de l'organe de cisaillement du système de la figure 7, vue d'en haut,
La figure 11 est une vue en perspective de détail de l'organe de cisaillement de la figure 10 assemblé dans le réservoir de fluide des figures 8 et 9, vue d'en haut,
La figure 12a est une vue en perspective de détail de l'organe de cisaillement de la figure 10 assemblé dans le réservoir de fluide des figures 8 et 9, vue d'en bas,
La figure 12b est une vue en perspective découpée similaire à celle de la figure 12a,
La figure 13 est une vue en perspective de détail de la clé de verrouillage du système de la figure 7,
La figure 14 est une vue en perspective de détail du corps intermédiaire du système de la figure 7,
La figure 15 est une vue schématique illustrant le cisaillement de fluide,
Les figures 16a et 16b sont des vues schématiques, respectivement en perspective éclatée et en perspective coupée, du système retardateur de la figure 7, dans la position des figures 1a et 1b,
Les figures 17a et 17b sont des vues similaires à celles des figures 16a et 16b, dans la position des figures 3a et 3b,
La figure 18 est une vue schématique de détail en section transversale d'une partie de l'autoinjecteur des figures 1 à 6, montrant plus particulièrement le système retardateur de la figure 7, dans la position des figures 1b et 2b,
Les figures 19a à 19c sont des vues schématique en coupe transversale, respectivement selon les plans de coupe A-A, B-B et C-C de la figure 18,
Les figures 20a et 20b sont des vues schématiques, respectivement de côté et en section transversale, d'un autoinjecteur selon un second mode de réalisation avantageux de la présente invention, en position de repos avant piquage,
Les figures 21a et 21b sont des vues similaires à celles des figures 20a et 20b, en position après piquage et avant injection,
Les figures 22a et 22b sont des vues similaires à celles des figures 21a et 21b, en position juste avant la fin d'injection et au moment du déclenchement du système retardateur,
Les figures 23a et 23b sont des vues similaires à celles des figures 22a et 22b, en début d'actionnement du système retardateur,
Les figures 24a et 24b sont des vues similaires à celles des figures 23a et 23b, en fin d'actionnement du système retardateur et avant actionnement du dispositif d'indication,
La figure 24c est une vue similaire à celle de la figure 24b selon un autre plan de coupe,
Les figures 25a à 25c sont des vues similaires à celles des figures 24a à 24c, en fin d'actionnement du dispositif d'indication et avant retrait de l'autoinjecteur du site d'injection,
La figure 26 est une vue similaire à celle de la figure 25a, en position de fin d'utilisation, après retrait de l'autoinjecteur du site d'injection,
La figure 27 est une vue en perspective éclatée du système retardateur selon ledit second mode de réalisation des figures 20 à 26,
Les figures 28 et 29 sont des vues en perspective de détail du système de la figure 27, montrant le sous-ensemble formé du réservoir, de l'organe de cisaillement et de l'élément indicateur,
La figure 30 est une vue en perspective de détail du réservoir et de l'élément indicateur du système de la figure 27,
Les figures 31a et 31b sont des vues schématiques partielles, respectivement en perspective éclatée et en perspective coupée, du système retardateur de la figure 27, dans la position des figures 22a et 22b,
Les figures 32a et 32b sont des vues similaires à celles des figures 31a et 31b, dans la position des figures 24a à 24c,
Les figures 33a et 33b sont des vues similaires à celles des figures 32a et 32b, dans la position des figures 25a à 25c,
La figure 34 est une vue schématique de détail en section transversale d'une partie de l'autoinjecteur des figures 20 à 26, montrant plus particulièrement le système retardateur de la figure 27, dans la position des figures 20b et 21b,
Les figures 35a à 35c sont des vues schématique en coupe transversale, respectivement selon les plans de coupe A-A, B-B et C-C de la figure 34.

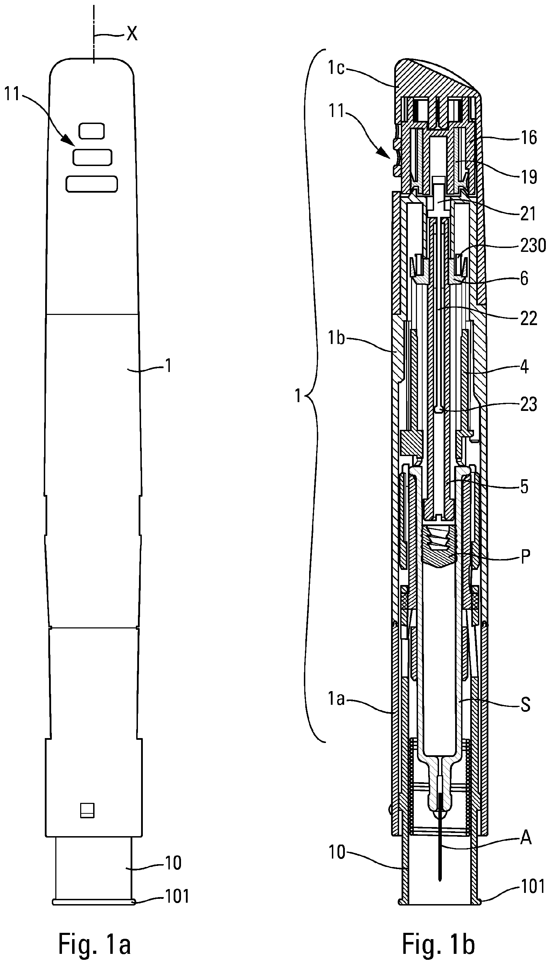

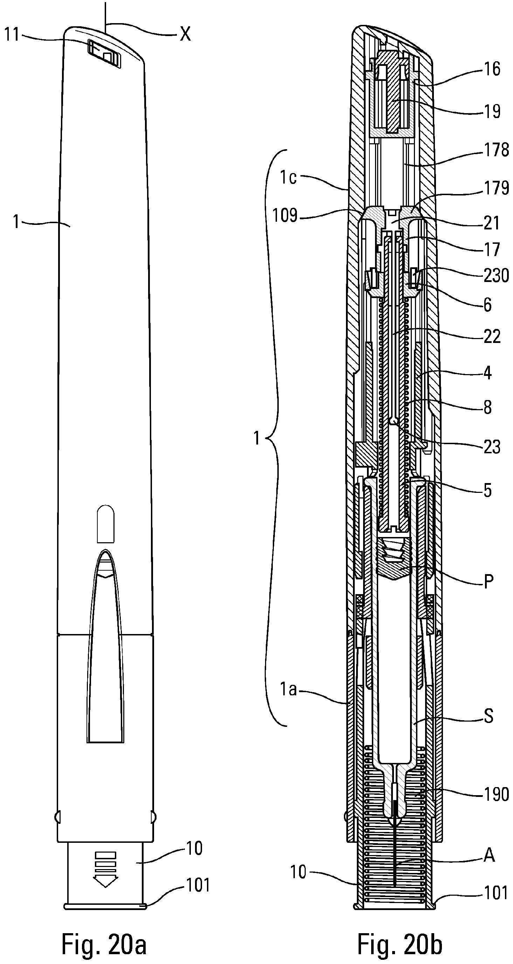

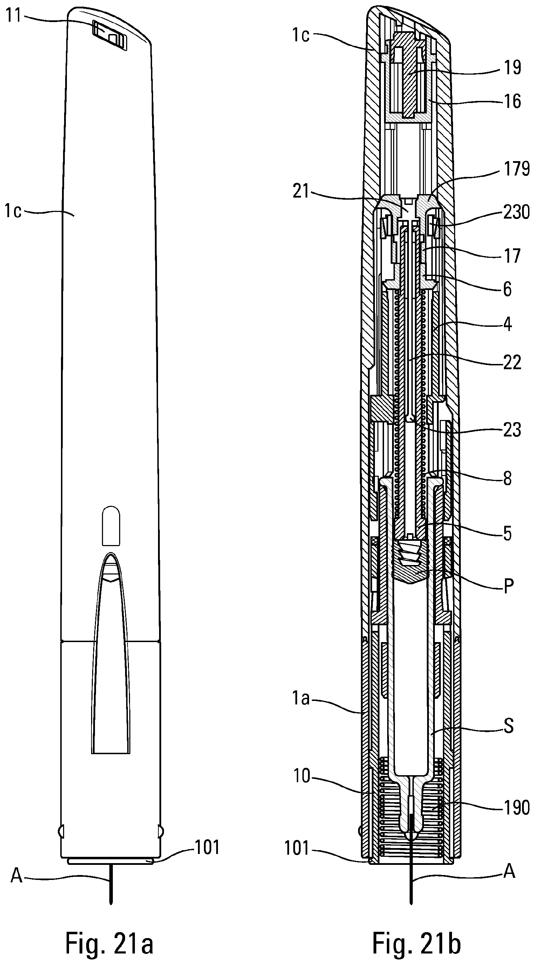

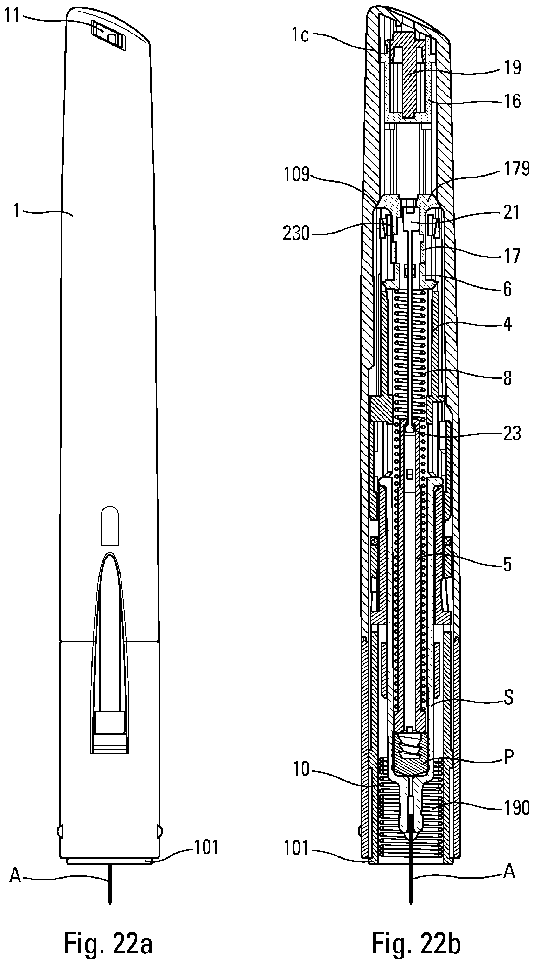

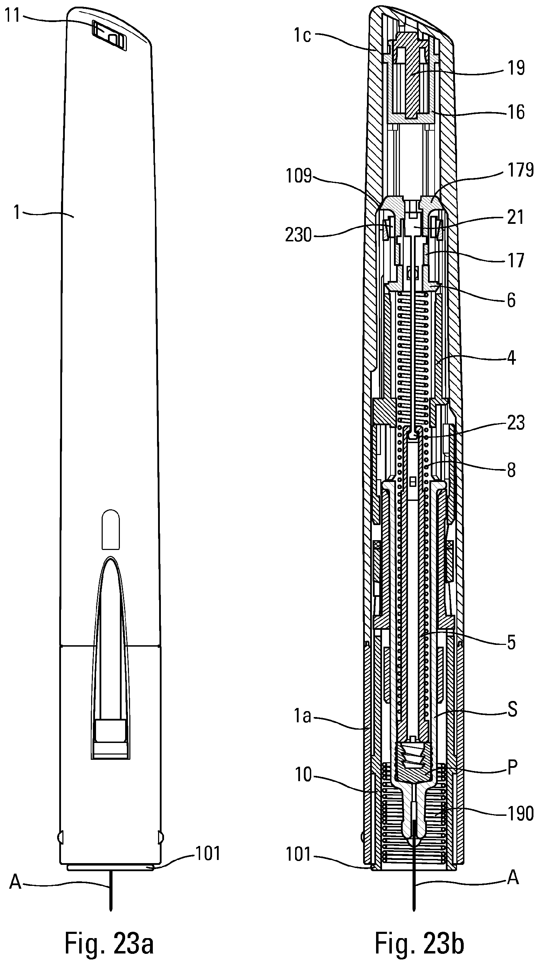

Dans la description ci-après, les termes "supérieur", "inférieur", "haut" et "bas" se réfèrent aux positions représentées sur les figures 1a à 6,15, 20a à 26 et 34. Les termes "axial" et "radial" se réfèrent à l'axe central longitudinal X, représenté notamment sur les figure 1a et 20a, qui correspond à l'axe longitudinal de l'aiguille.

L'autoinjecteur va être décrit ci-après en référence à deux modes de réalisation avantageux de celui-ci. Il est toutefois à noter que les autoinjecteurs, qui sont des appareils complexes, comprennent plusieurs modules pour réaliser plusieurs fonctions. Ces divers modules peuvent être utilisés séparément et indépendamment les uns des autres, sans être nécessairement combinés aux autres modules, et pourraient notamment être utilisés dans des autoinjecteurs de forme différente de celle représentée sur les dessins. De plus, il est à noter que les dessins sont des vues schématiques qui ne représentent pas forcément la forme exacte des composants d'un autoinjecteur, et ne sont pas forcément à l'échelle, notamment dans des buts de clarté. Par ailleurs, les dessins ne représentent pas forcément tous les éléments constitutifs d'un autoinjecteur, mais seulement les éléments nécessaires au fonctionnement de la présente invention. Ainsi, divers éléments et modules supplémentaires et/ou complémentaires pourraient être associés à l'autoinjecteur représenté sur les figures.

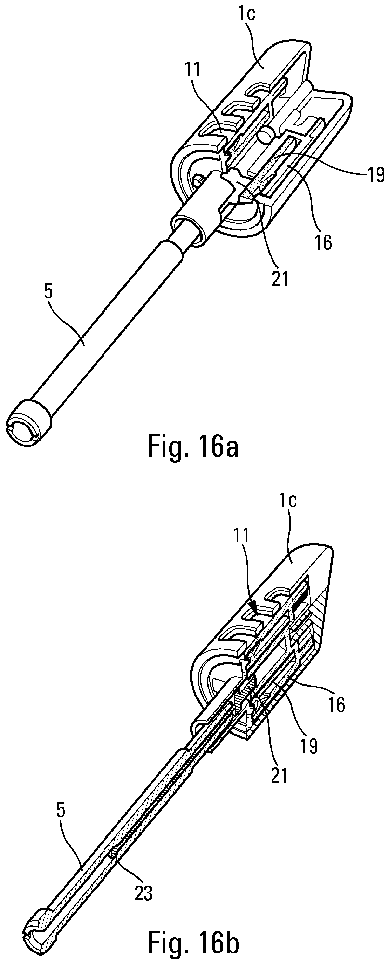

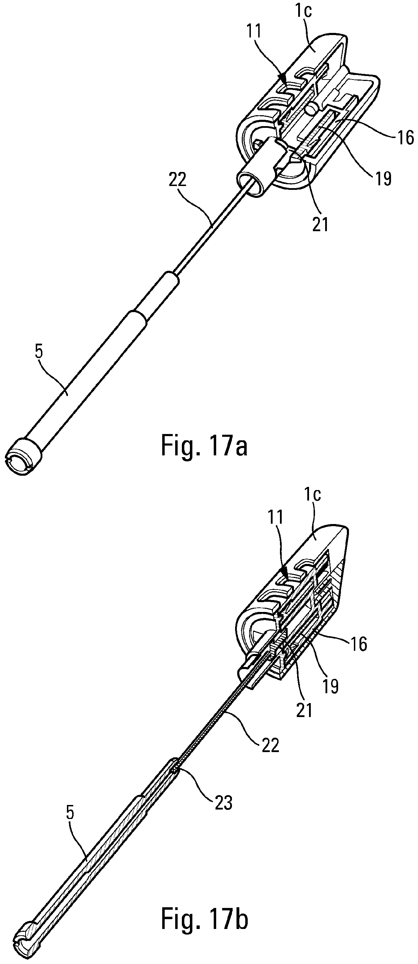

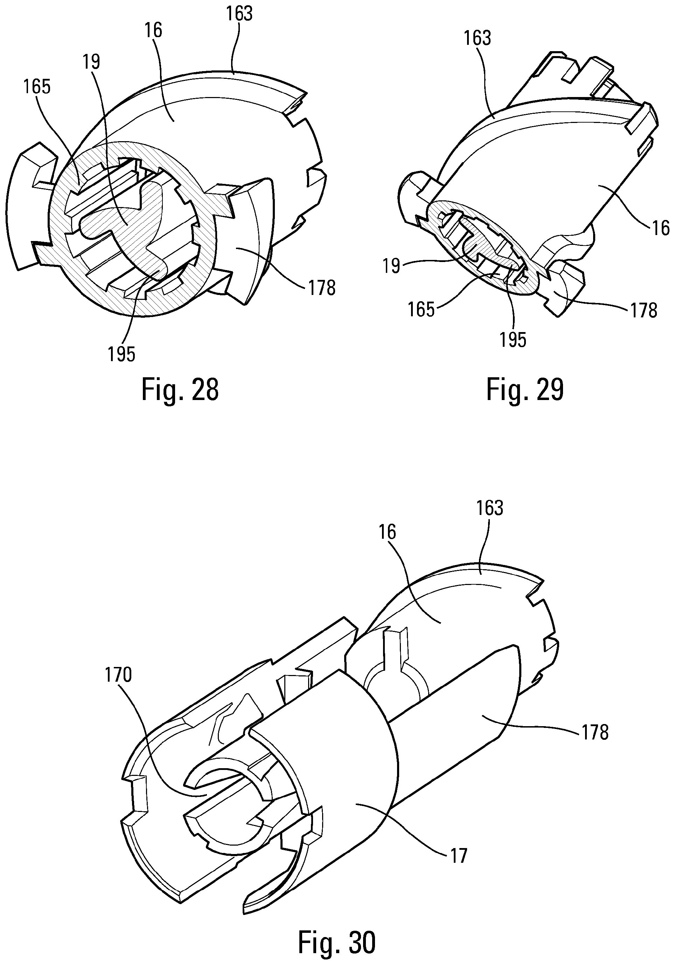

L'autoinjecteur représenté sur les figures comporte un corps 1 dans lequel coulisse axialement un manchon actionneur 10, dont l'extrémité inférieure 101 est destinée à venir en contact avec le corps du patient autour de la zone d'injection. Dans l'exemple des figures 1 à 19, l'autoinjecteur comporte un corps inférieur 1a, un corps intermédiaire 1b et un corps supérieur 1c qui sont assemblés pour former le corps 1 de l'autoinjecteur. Dans l'exemple des figures 20 à 35, l'autoinjecteur comporte un corps inférieur 1a et un corps supérieur 1c qui sont assemblés pour former le corps 1 de l'autoinjecteur. Ci-après, le terme "corps" et la référence numérique "1" seront utilisés pour désigner ledit corps unitaire formé par l'assemblage dudit corps inférieur 1a avec ledit corps intermédiaire 1b et/ou ledit corps supérieur 1c. Il est à noter que le corps 1 pourrait être formé d'un nombre quelconques de parties de corps, et que les modes de réalisation des figures, avec deux ou trois parties de corps, ne sont pas limitatifs.

Un réservoir S peut être inséré dans ledit corps 1 de l'autoinjecteur, ledit réservoir S étant de préférence fixe dans ledit corps 1. Ce réservoir S contient du produit fluide, et comporte un piston P et une aiguille A. Le piston P est adapté à se déplacer dans ledit réservoir S pour injecter le produit fluide à travers ladite aiguille A. Eventuellement, la présente invention pourrait aussi s'appliquer à un réservoir sans aiguille, notamment dans un dispositif d'injection sans aiguille.

La présente description sera faite en référence à une seringue S, qui peut être de tout type. Plus généralement, il est entendu que le terme "seringue" dans la présente description englobe tout type de réservoir associé à une aiguille. De préférence, le réservoir S est une seringue pré-remplie.

Avant actionnement de l'autoinjecteur, l'aiguille A de la seringue S peut être protégée par un capuchon (non représenté), l'autoinjecteur pouvant comporter un capot (non représenté) que l'utilisateur peut retirer avant actionnement. Le retrait du capot provoque avantageusement le retrait du capuchon.

Avant actionnement, le manchon actionneur 10 est dans une première position projetée dans laquelle il entoure l'aiguille A, comme représenté sur les figures 1a et 1b d'une part et 20a et 20b d'autre part. Lors de l'actionnement, le manchon actionneur 10 coulisse à l'intérieur du corps 1 vers une position d'actionnement, pour exposer l'aiguille A et permettre le piquage puis l'injection du produit fluide.

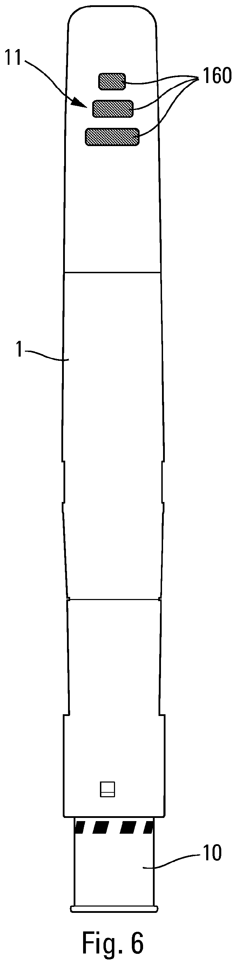

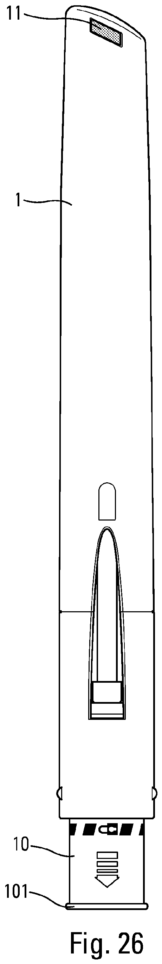

Après l'injection, lorsque l'utilisateur retire l'autoinjecteur du site d'injection, le manchon actionneur 10 revient dans une seconde position projetée de fin d'utilisation, dans laquelle il est à nouveau disposé autour de l'aiguille A, pour éviter tout risque de blessure avec ladite aiguille, comme représenté sur les figures 6 et 26.

Le manchon actionneur 10 est avantageusement sollicité vers ses positions projetées par un organe élastique ou ressort 190 (visibles sur les figures 20b, 21b, 22b, 23b, 24b, 24c, 25b et 25c du second mode de réalisation des figures 20 à 35, mais non représenté sur le premier mode de réalisation des figures 1 à 19), qui peut être de type quelconque. Avantageusement, dans ladite position de fin d'utilisation, ledit manchon actionneur 10 est verrouillé, et ne peut plus être déplacé axialement vers l'intérieur dudit corps 1. Ce verrouillage peut par exemple être réalisé par des pattes (non représentées), solidaires du corps 1 ou du réservoir S, et coopérant avec des ouvertures (non représentées) dudit manchon actionneur 10 lorsque celui-ci atteint sa seconde position projetée. Ce verrouillage, qui n'est pas essentiel au fonctionnement de la présente invention, ne sera pas décrit plus en détail ci-après. Il pourrait être réalisé différemment du mode de réalisation particulier mentionné ci-dessus. En particulier, il pourrait être réalisé conformément aux enseignements des documents WO2013175140 ou WO2013175142.

L'autoinjecteur comporte également un système d'injection automatique, comportant notamment une tige de piston 5 adaptée à coopérer avec le piston P pour le déplacer dans le réservoir S pour distribuer le produit fluide à travers l'aiguille A. Cette tige de piston 5 est classiquement sollicitée par un ressort d'injection 8 vers sa position de distribution, et retenue avant actionnement dans sa position de repos par une serrure d'injection appropriée. Le ressort d'injection 8 n'est représenté que sur les figures 20 à 35 du second mode de réalisation.

Une serrure d'injection avantageuse est notamment décrite dans le document WO2015155484.

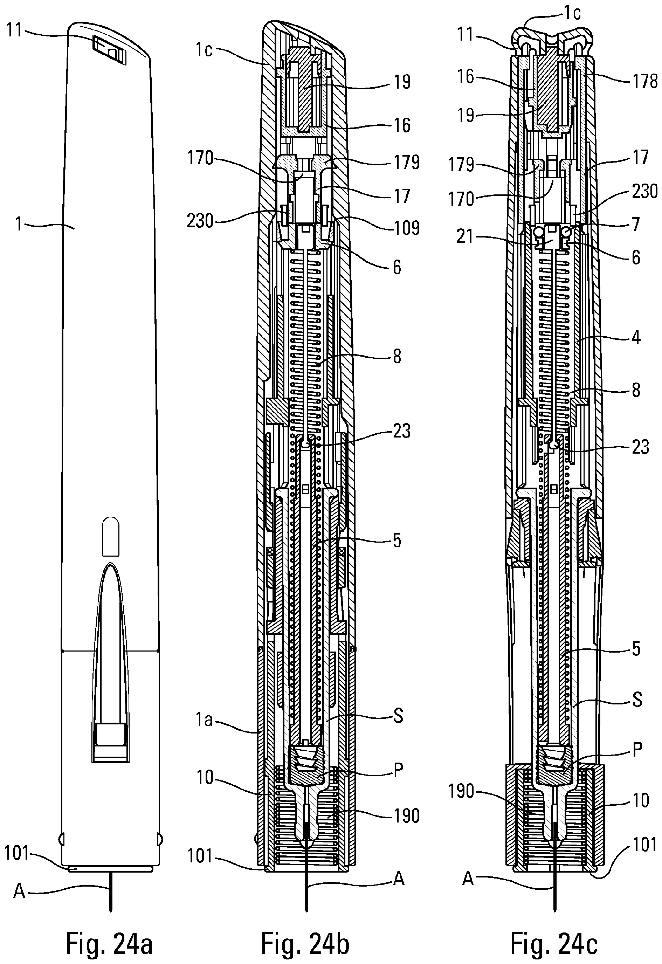

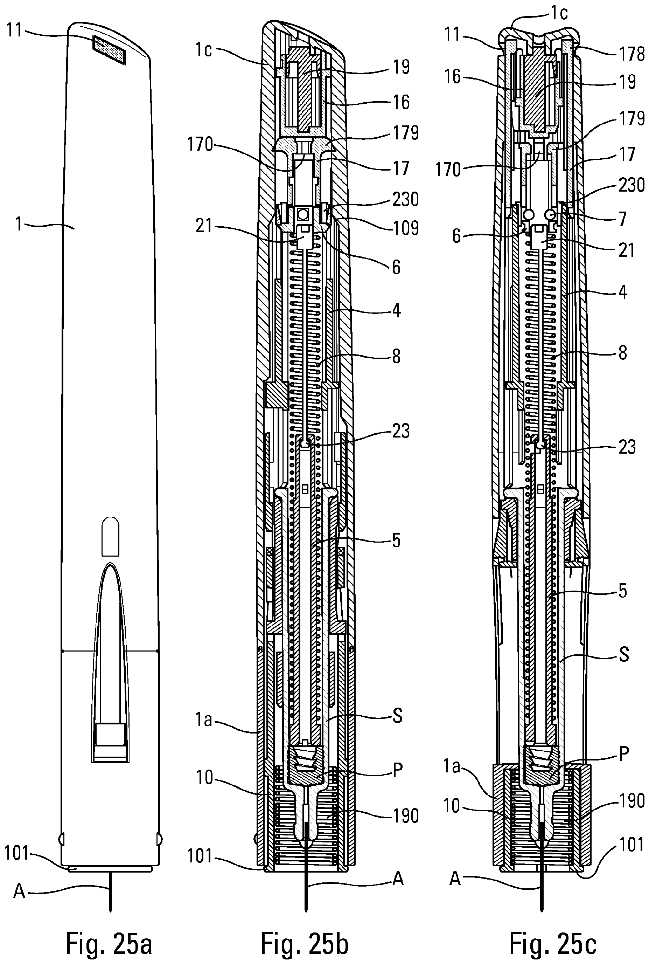

La serrure peut comprendre au moins un élément de blocage 7, retenu en position de blocage par une bague de blocage 230 fixée, notamment encliquetée sur un organe support 6 sur lequel s'appuie le ressort d'injection 8. Il est à noter que ledit au moins un élément de blocage 7 n'est visible que sur les figures 24c et 25c du second mode de réalisation des figures 20 à 35. Le déclenchement de ladite serrure d'injection provoque l'actionnement desdits moyens d'injection et donc l'injection du produit fluide à travers l'aiguille. Ladite serrure d'injection peut en outre comporter un manchon de commande 4 disposé dans ledit corps 1, ledit manchon de commande 4 contenant ladite tige de piston 5 et ledit ressort d'injection 8, ladite tige de piston 5 comportant un évidement radial recevant au moins un élément de blocage mobile entre une position de blocage et une position de déblocage. Ledit au moins un élément de blocage 7 est de préférence de forme sensiblement sphérique, tel qu'une bille. Avantageusement, lesdites billes sont sollicitées radialement vers l'extérieur par ladite tige de piston 5 et sont retenues en position de blocage par la bague de blocage 230. Cette bague de blocage 230 est déplaçable axialement par rapport à ladite tige de piston 5 et par rapport audit organe support 6 entre une position de verrouillage, dans laquelle elle maintient lesdites billes en position de blocage, et une position de déverrouillage, dans laquelle lesdites billes sont libérés pour ainsi débloquer ladite serrure d'injection, permettant audit ressort d'injection 8 de déplacer ladite tige de piston 5 vers sa position d'injection. La bague de blocage 230 peut notamment être déplacée vers sa position de déverrouillage par ledit manchon de commande 4.

Lorsque l'aiguille A de la seringue S a pénétré le corps de l'utilisateur, la bague de blocage 230 est déplacée axialement vers le haut, ce qui a pour effet de libérer les billes de leur position de blocage, celles-ci étant alors déplacées radialement vers l'extérieur. La tige de piston 5 n'est alors plus retenue par les billes et elle est donc déplacée axialement vers le bas pour réaliser l'injection du produit.

L'autoinjecteur comporte un dispositif d'indication visuel, sonore et/ou tactile pour indiquer à l'utilisateur, notamment par un son audible, par une vibration et/ou par une indication visuelle et/ou tactile, qu'il peut retirer l'autoinjecteur du site d'injection. Ledit dispositif d'indication visuel, sonore et/ou tactile est disposé de préférence à l'extrémité arrière dudit corps 1, opposée audit site d'injection. En particulier, dans les exemples représentés, le dispositif d'indication comporte un élément indicateur qui donne une indication visuelle, par un affichage 160 adéquat dans une ou plusieurs fenêtre(s) 11 du corps 1. Avantageusement une indication sonore et/ou tactile peut également être fournie, comme cela sera décrit plus en détails ci-après.

Pour éviter que l'utilisateur ne retire l'autoinjecteur du site d'injection dès la fin d'injection, l'autoinjecteur comporte un système retardateur, qui va retarder l'actionnement dudit dispositif d'indication par rapport à la fin de l'injection.

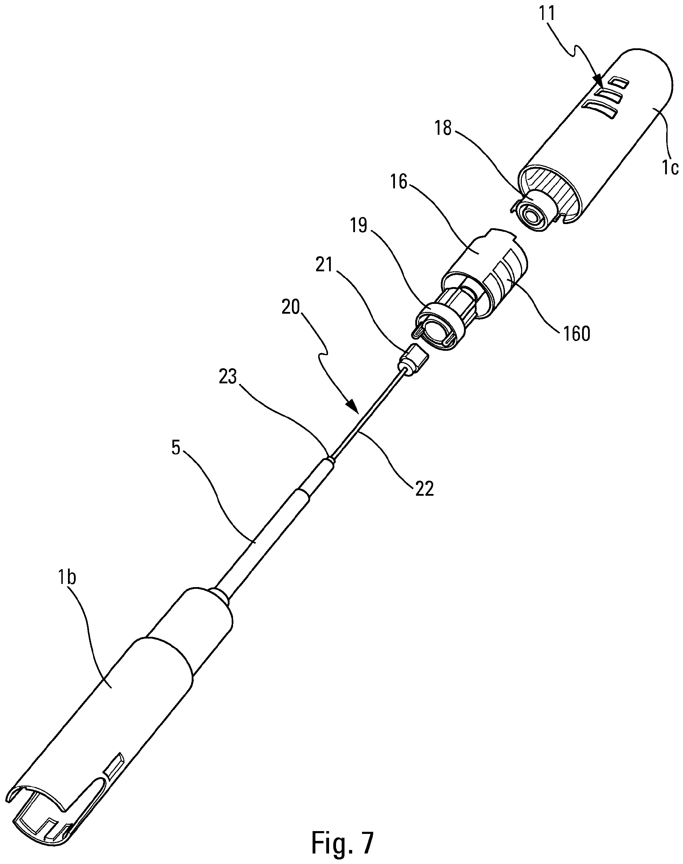

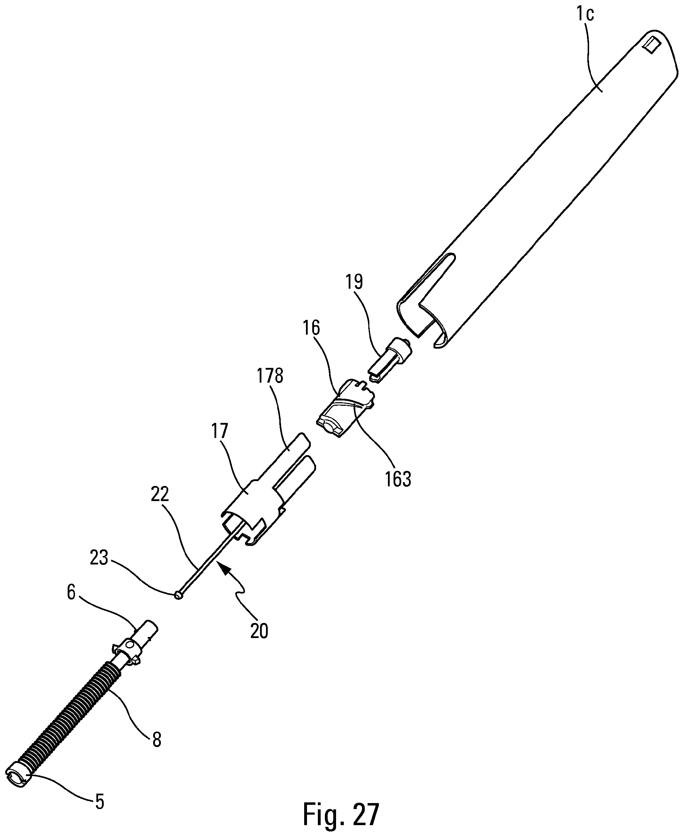

Les figures 7 à 19 illustrent un système retardateur selon un premier mode de réalisation avantageux, et les figures 27 à 35 illustrent un système retardateur selon un second mode de réalisation avantageux.

Ce système retardateur a principalement pour but de décaler dans le temps l'indication visuelle, sonore et/ou tactile après la fin de l'injection du produit fluide à l'intérieur dudit corps. Ceci permet notamment une diffusion pendant quelques secondes du produit après son injection. Un tel retardateur procure aussi un bénéfice pour l'utilisateur, qui n'a plus à compter, par exemple jusqu'à 10, après son injection, le temps pris pour ce comptage pouvant être très variable d'un utilisateur à un autre. Avec un retardateur, on facilite la séquence d'utilisation d'un autoinjecteur.

Le retardateur mécanique permet de décaler ainsi de quelques secondes cette indication de fin d'utilisation par rapport à la fin de l'injection, ce retard étant prédéterminable.

L'invention exploite le phénomène de cisaillement de fluide pour générer ledit retard, et utilise un réservoir 16, un organe de cisaillement 19 disposé dans ledit réservoir 16, et un fluide disposé dans ledit réservoir 16, autour dudit organe de cisaillement 19.

Dans les exemples représentés, l'organe de cisaillement 19 est fixe en rotation par rapport au corps 1, et le réservoir 16 est mobile en rotation par rapport audit corps 1. L'inverse est toutefois aussi envisageable.

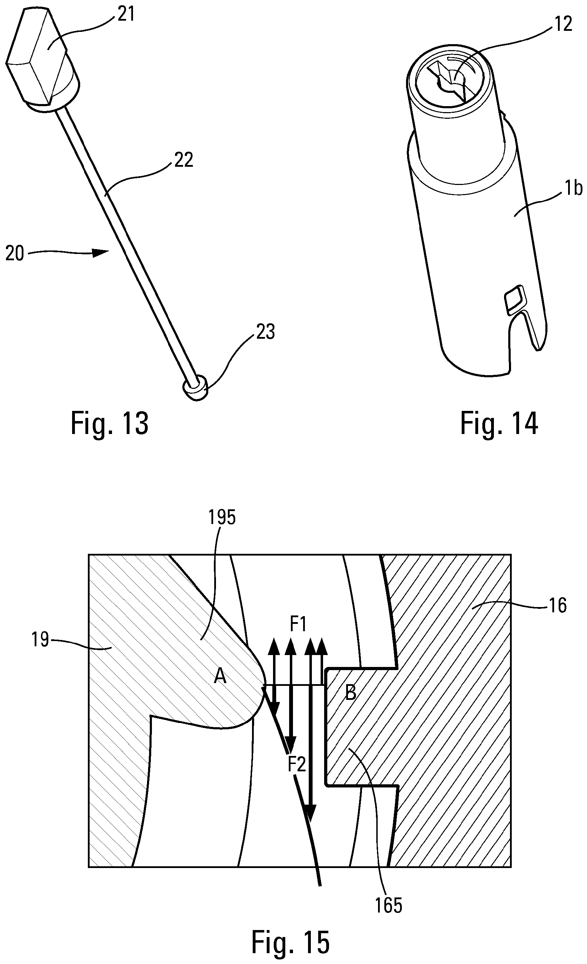

Un fluide est disposé dans ledit réservoir 16, autour dudit organe de cisaillement 19.Le réservoir 16 comporte des projections 165 sur sa surface interne et l'organe de cisaillement 19 comporte des projections 195 sur sa surface externe. Ces projections 165, 195 génèrent des restrictions à l'écoulement du fluide. Une rotation dudit réservoir 16 par rapport audit organe de cisaillement 19 implique donc un cisaillement du fluide, en particulier lorsqu'une projection 165 du réservoir 16 se trouve face à une projection 195 de l'organe de cisaillement 19, comme visible sur la figure 15.

Le terme "cisaillement de fluide" est un phénomène de viscosité dynamique. La viscosité dynamique correspond à la contrainte de cisaillement qui accompagne l'existence d'un gradient de vitesse d'écoulement dans la matière. Lorsque la viscosité augmente, la capacité du fluide à s'écouler diminue.

On exploite également le phénomène de couche limite, qui est lié à la viscosité dynamique. La couche limite est la zone d'interface entre un corps et le fluide environnant lors d'un mouvement relatif entre les deux, conséquence de sa viscosité. Lorsqu'un fluide s'écoule le long d'une paroi supposée fixe, les vitesses sur la paroi sont nulles alors qu'à l'infini (c'est-à-dire loin de l'obstacle) elles sont égales à la vitesse de l'écoulement non perturbé. La loi de variation dépend de la viscosité du fluide qui induit un frottement entre les couches voisines : la couche la plus lente tend à freiner la couche la plus rapide qui, en retour, tend à l'accélérer. Dans ces conditions, une forte viscosité égalise au maximum les vitesses. Au contraire, si le fluide est peu visqueux, les différentes couches sont beaucoup plus indépendantes : la vitesse à l'infini se maintient jusqu'à une courte distance de l'obstacle et il y a une variation plus forte des vitesses dans la petite épaisseur de la couche limite.

En référence à la figure 15, qui s'applique aux deux modes de réalisation de l'invention, le point A (projection 195) est fixe alors que le point B (projection 165) se déplace. Au contact du point A, la vitesse du fluide est donc nulle alors qu'au contact du point B, elle est maximale. Les flèches F1 illustrent la force due à la viscosité dynamique qui provient du gradient de vitesse. Les flèches F2 illustrent la vitesse du fluide en fonction de la distance entre les points A (organe de cisaillement 19 fixe) et B (réservoir 16 mobile). La viscosité du fluide et la distance entre les points A et B sont donc des paramètres importants pour prédéterminer l'effet de cisaillement du fluide, et ainsi son effet de freinage sur la rotation du réservoir 16.

Selon la viscosité du fluide contenu dans le réservoir 16 et/ou selon la forme et/ou les dimensions des profils 165, 195 du réservoir 16 et de l'organe de cisaillement 19, on peut régler assez précisément ledit freinage et donc le temps entre le moment où le système retardateur est déclenché, en fin d'injection, et le moment où le réservoir 16 aura réalisé sa rotation prédéfinie pour réaliser l'indication, et notamment indiquer dans la fenêtre de l'indicateur que l'autoinjecteur peut être retiré du site d'injection. L'actionnement du dispositif d'indication visuel, sonore et/ou tactile est donc retardée par rapport à la fin de l'injection, ce qui permet au produit injecté de se diffuser dans le site d'injection pendant cette durée de retard.

La figure 7 illustre une vue schématique en perspective éclatée de ce système retardateur selon un premier mode de réalisation avantageux. Celui-ci comprend le corps supérieur 1c, un ressort retardateur 18, de préférence réalisé sous la forme d'un ressort à spirale, le réservoir 16 contenant un fluide approprié, l'organe de cisaillement 19 disposé dans ledit réservoir 16, une clé de verrouillage 20, la tige de piston 5 et le corps intermédiaire 1b.

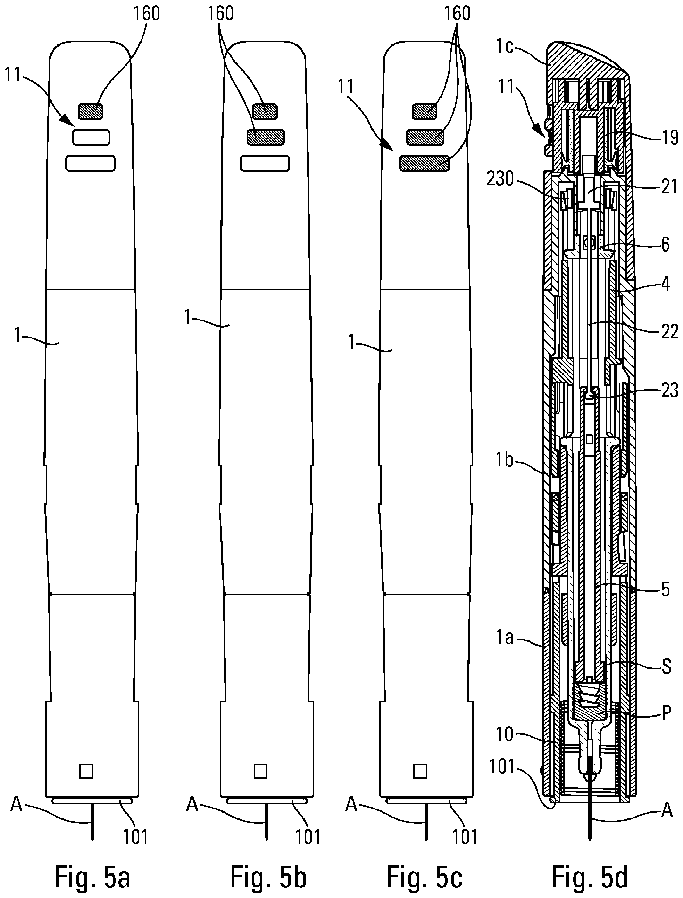

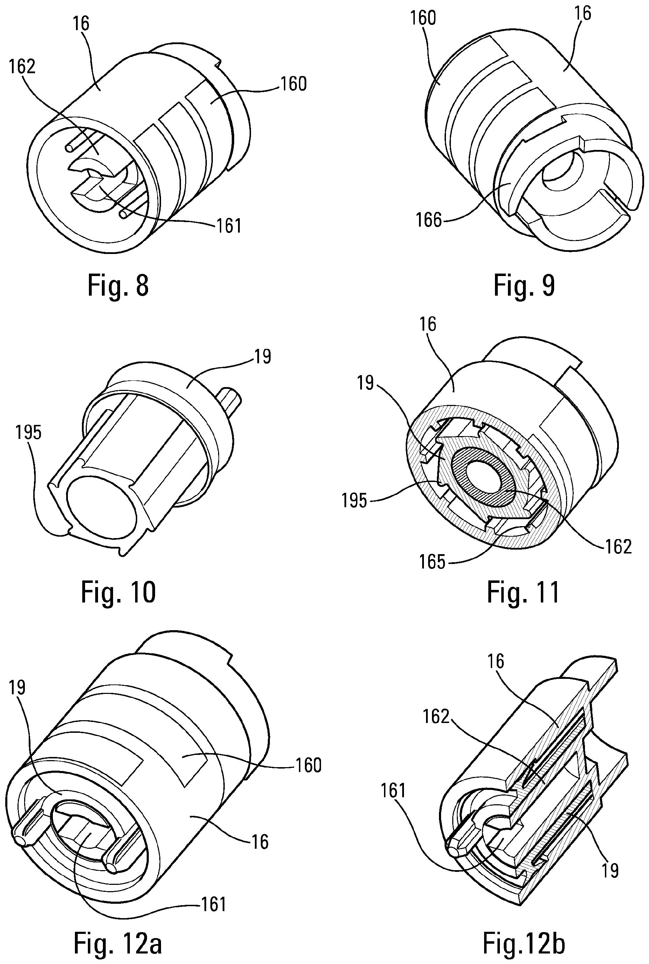

Dans l'exemple représenté, le réservoir 16 forme également l'élément indicateur du dispositif d'indication visuel, sonore et/ou tactile. Avantageusement, ledit réservoir 16 peut comporter un affichage 160 approprié pour venir indiquer la fin d'utilisation de l'autoinjecteur dans une ou plusieurs fenêtres du corps 1, notamment du corps supérieur 1c. Dans l'exemple représenté sur les figures 1 à 19, le corps 1 comporte trois fenêtres 11 qui affichent la progression de l'actionnement du système retardateur. Ainsi, les figures 5a à 5c montrent l'affichage progressif du marquage 160 du réservoir 16 dans les fenêtres 11 du corps 1. Les figures 8, 9 et 12a illustrent ce marquage 160 réalisé sur la surface externe dudit réservoir 16.

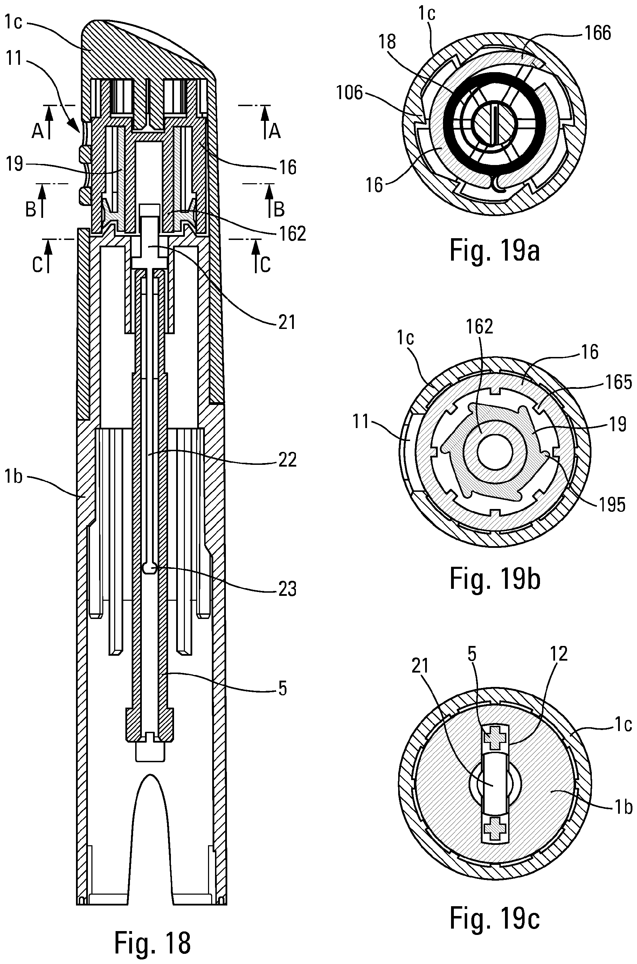

Le ressort à spirale 18 est fixé d'une part au corps supérieur 1c et d'autre part au réservoir 16, comme visible notamment dans la figure 19a. En variante, le ressort à spirale pourrait être fixé à une autre partie du corps 1, par exemple le corps intermédiaire 1b, ou à tout élément fixé audit corps 1. En variante, ledit ressort à spirale pourrait être fixé à l'organe de cisaillement 19, auquel cas c'est le réservoir 16 qui serait fixe en rotation par rapport au corps 1. Dans cette variante, cet organe de cisaillement mobile en rotation pourrait former l'élément indicateur du dispositif d'indication visuel, sonore et/ou tactile.

La clé de verrouillage 20 comporte une tête 21 adaptée à coopérer avec le système retardateur, une tige longitudinale 22, et en embout 23 adapté à coopérer avec la tige de piston 5.

En position avant déclenchement du système retardateur, la tête 21 de la clé de verrouillage 20 est en position de blocage dans laquelle elle coopère avec un profil correspondant 12 du corps intermédiaire 1b et avec un profil correspondant 161 du réservoir 16, de sorte que celui-ci est bloqué en rotation par ladite clé. Lorsque la tige de piston 5 arrive vers sa position de fin d'injection, elle va coopérer avec l'embout 23 de la clé de verrouillage 20, et ainsi tirer ladite clé axialement vers le bas. De ce fait, la tête 21 de ladite clé de verrouillage 20 sort axialement dudit profil 161 du réservoir 16, de sorte que ledit réservoir 16 n'est plus bloqué en rotation par ladite clé 20. Avantageusement, ledit profil 161 du réservoir est formé sur un manchon interne 162 du réservoir 16, disposé à l'intérieur dudit organe de cisaillement 19, comme visible notamment sur les figures 8, 11, 12, 18 et 19b.

Le ressort à spirale 18 sollicite le réservoir 16 en rotation. Tant que le réservoir 16 est bloqué par ladite clé de verrouillage 20, le système de retardement est donc également bloqué.

Lorsque le système retardateur est déclenché, le ressort 18 sollicite en rotation le réservoir 16. Celui-ci subit un couple de freinage dû au cisaillement du fluide contenu entre la paroi du réservoir 16 et l'organe de cisaillement 19. La rotation du réservoir 16 est donc freinée par ledit fluide.

Avantageusement, le réservoir 16 peut comporter une patte flexible 166, visible sur les figures 9 et 19a, dont l'extrémité génère un bruit pendant toute la rotation dudit réservoir 16, par exemple sur des profils 106 appropriés du corps supérieur 1c, et fournissant ainsi une indication sonore du fonctionnement du système retardateur: lorsque le bruit s'arrête, l'actionnement du système retardateur et celui du dispositif d'indication sont terminés, et l'utilisateur peut retirer l'autoinjecteur du site d'injection. Bien entendu, cette patte 166 peut simultanément aussi fournir un freinage supplémentaire de la rotation. En variante, on peut prévoir plusieurs pattes flexibles 166, par exemple deux. Dans une autre variante, on peut aussi prévoir un seul profil 106 sur le corps supérieur 1c, générant un bruit avec la patte flexible 166 seulement en toute fin d'actionnement du dispositif d'indication, ce qui fournit une indication sonore de fin d'utilisation: lorsque le bruit est généré, l'actionnement du système retardateur et celui du dispositif d'indication sont terminés, et l'utilisateur peut retirer l'autoinjecteur du site d'injection.

La figure 27 illustre une vue schématique en perspective éclatée de ce système retardateur selon un second mode de réalisation avantageux. Celui-ci comprend le corps supérieur 1c, le réservoir 16 contenant un fluide approprié, l'organe de cisaillement 19 disposé dans ledit réservoir 16, un élément indicateur 17 dudit dispositif d'indication visuel, sonore et/ou tactile, une clé de verrouillage 20, la tige de piston 5, l'organe support 6 et le ressort d'injection 8.

Dans ce mode de réalisation, le corps 1 n'est composé que de deux parties, une partie de corps inférieur 1a et une partie de corps supérieur 1c. Il n'y a donc pas dans ce mode de réalisation de partie de corps intermédiaire.

L'élément indicateur 17 est déplaçable axialement dans ledit corps 1, mais n'est pas rotatif. Le réservoir 16 est lui rotatif mais non déplaçable axialement, et il comporte sur sa surface externe au moins un filetage externe 163, qui coopère avec l'élément indicateur 17. Ainsi, un déplacement axial dudit élément indicateur 17 autour dudit réservoir 16 va provoquer une rotation dudit réservoir 16 autour dudit organe de cisaillement 19, qui dans cet exemple est fixe par rapport au corps 1. En variante, on pourrait envisager l'inverse, à savoir un élément indicateur 17 pourvu d'un filetage, une translation de ce filetage par rapport au réservoir provoquant la rotation dudit réservoir.

La clé de verrouillage 20 comporte une tête 21 adaptée à coopérer avec le système retardateur, une tige longitudinale 22, et en embout 23 adapté à coopérer avec la tige de piston 5.

En position avant déclenchement du système retardateur, la tête 21 de la clé de verrouillage 20 est en position de blocage dans laquelle elle coopère avec un évidement axial 170 de l'élément indicateur 17, de sorte que celui-ci est bloqué en translation par ladite clé. Lorsque la tige de piston 5 arrive vers sa position de fin d'injection, elle va coopérer avec l'embout 23 de la clé de verrouillage 20, et ainsi tirer ladite clé axialement vers le bas. De ce fait, la tête 21 de ladite clé de verrouillage 20 sort axialement dudit évidement 170, de sorte que ledit élément indicateur 17 n'est plus bloqué en translation par ladite clé 20.

Le ressort d'injection 8 sollicite l'élément indicateur 17 axialement en translation en direction de l'arrière dudit corps supérieur 1c. Tant que l'élément indicateur 17 est bloqué par ladite clé de verrouillage 20, le dispositif d'indication visuel, sonore et/ou tactile est donc également bloqué.

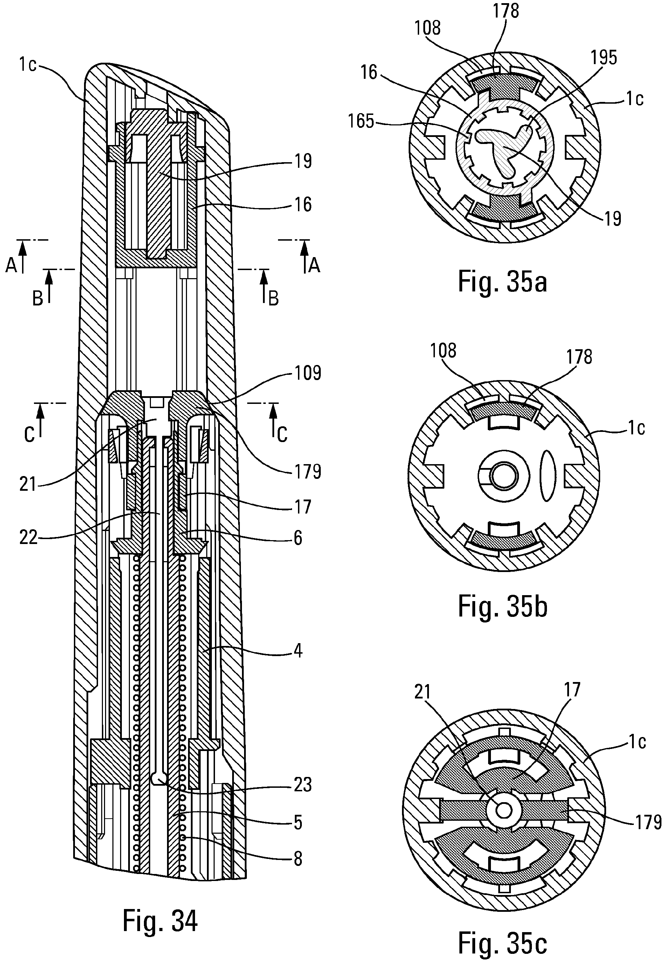

Dans l'exemple représenté, avant actionnement du système retardateur, l'élément indicateur 17 est bloqué contre tout déplacement axial vers le haut par une paroi tronconique ou inclinée 109 du corps supérieur 1c qui coopère avec des pattes flexibles 179 dudit élément indicateur 17. Dans l'exemple représenté, il y a deux pattes flexibles 179 diamétralement opposées, mais on pourrait envisager un nombre différent de pattes flexibles, par exemple une seule ou plus de deux pattes. Comme visible notamment sur la figure 34, en position de blocage, ladite tête 21 de la clé de verrouillage 20 empêche la déformation radiale vers l'intérieur desdites pattes flexibles 179 de l'élément indicateur 17. Lorsque ladite tête 21 n'est plus en position de blocage, lesdites pattes flexibles 179 peuvent se déformer radialement vers l'intérieur, sous l'effet du ressort d'injection 8 qui pousse ledit élément indicateur 17 axialement vers le haut, et du fait de la coopération desdites pattes flexibles 179 avec la paroi inclinée 109 du corps 1.

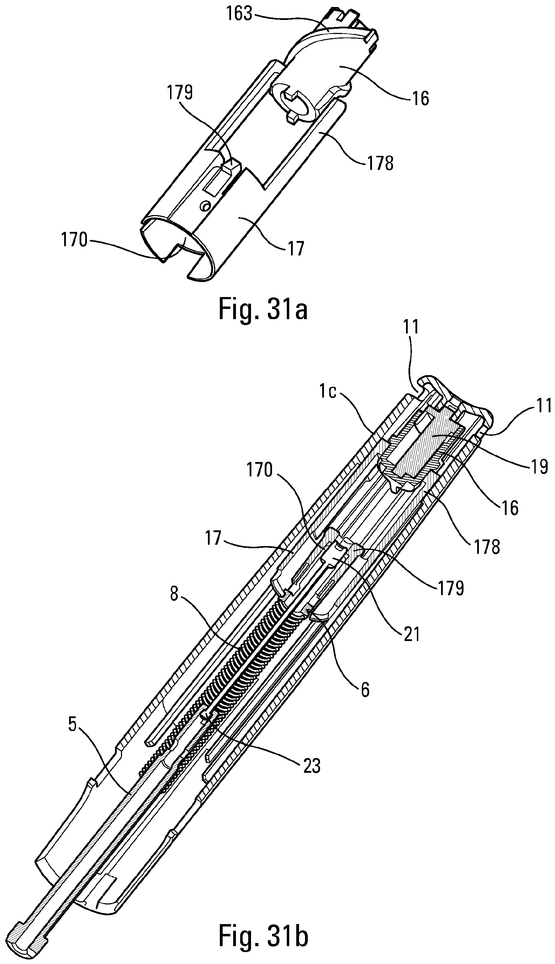

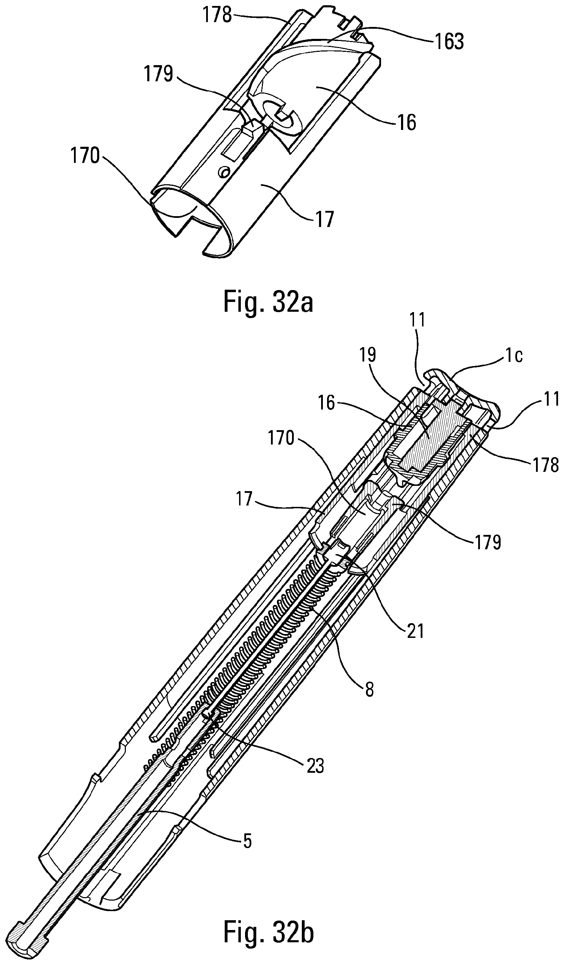

Lorsque ladite clé de verrouillage 20 libère ledit élément indicateur 17 en fin d'injection, celui-ci est donc déplacé axialement par ledit ressort d'injection 8. Le système retardateur est alors déclenché, avec ledit l'élément indicateur 17 coopérant avec ledit réservoir 16 pour le faire tourner autour dudit organe de cisaillement 19. Dans l'exemple représenté, cette coopération se fait entre des pattes rigides 178 de l'élément indicateur 17 et les filetages externes 163 du réservoir 16, comme illustré sur les figures 31a, 31b et 32a, 32b. Ces pattes rigides 178 coulissent dans des gorges 108 du corps supérieur 1c, ce qui empêche toute rotation dudit élément indicateur 17. La vitesse de déplacement axial de l'élément indicateur 17 correspond donc à la vitesse de rotation dudit réservoir 16, qui subit un freinage dû au cisaillement du fluide contenu entre la paroi du réservoir 16 et l'organe de cisaillement 19. Le principe du cisaillement de fluide, illustré sur la figure 15, est le même que pour le premier mode de réalisation des figures 1 à 19.

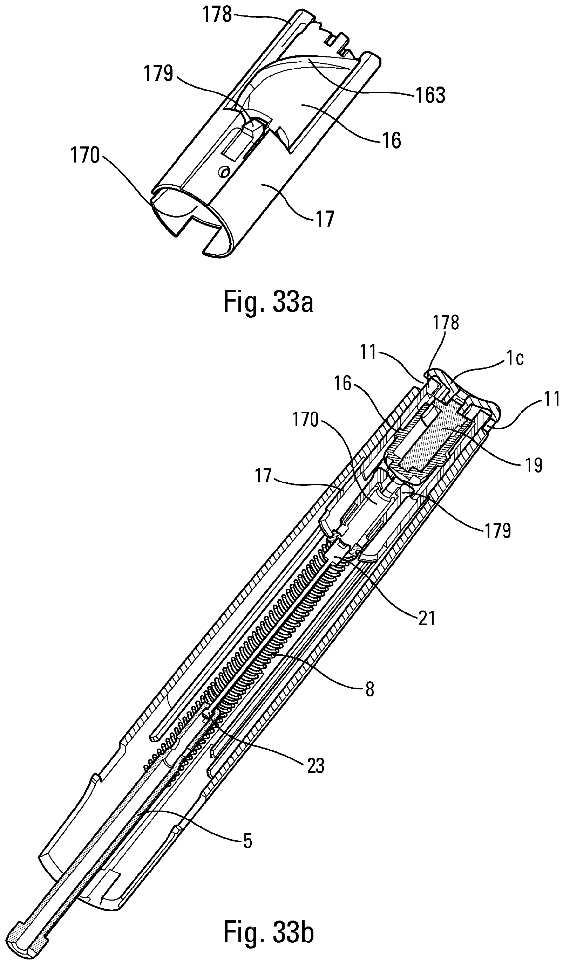

Lorsque lesdites pattes rigides 178 de l'élément indicateur 17 ne coopèrent plus avec les filetages externes 163 du réservoir 16, le déplacement axial dudit élément indicateur 17 n'est plus freiné, et celui-ci est donc projeté par le ressort d'injection 8 dans sa position d'indication, comme illustré sur les figures 33a et 33b, où les pattes rigides 178 sont disposées face aux fenêtres d'indication 11 du corps 1. Ce déplacement non freiné ou projection de l'élément indicateur 17 permet de générer une indication sonore, par contact entre l'élément indicateur 17 et le corps 1. Cette indication sonore peut être réalisée au niveau desdites pattes rigides ou sur une autre partie de l'élément indicateur 17 adaptée à coopérer avec une partie appropriée du corps 1. Il est à noter que le choc générant l'indication sonore peut aussi fournir une indication tactile en générant une vibration de l'autoinjecteur dans la main de l'utilisateur, utile notamment pour les malentendants.

Dans les deux modes de réalisation décrits ci-dessus, le fluide utilisé dans le système retardateur peut être de tout type approprié, par exemple une graisse.

Le dispositif retardateur permet donc de décaler d'un temps prédéterminé le moment où l'indicateur va indiquer la fin d'utilisation, à partir du moment où la phase d'injection est terminée.

Une phase complète d'actionnement de l'autoinjecteur va être décrite ci-après.

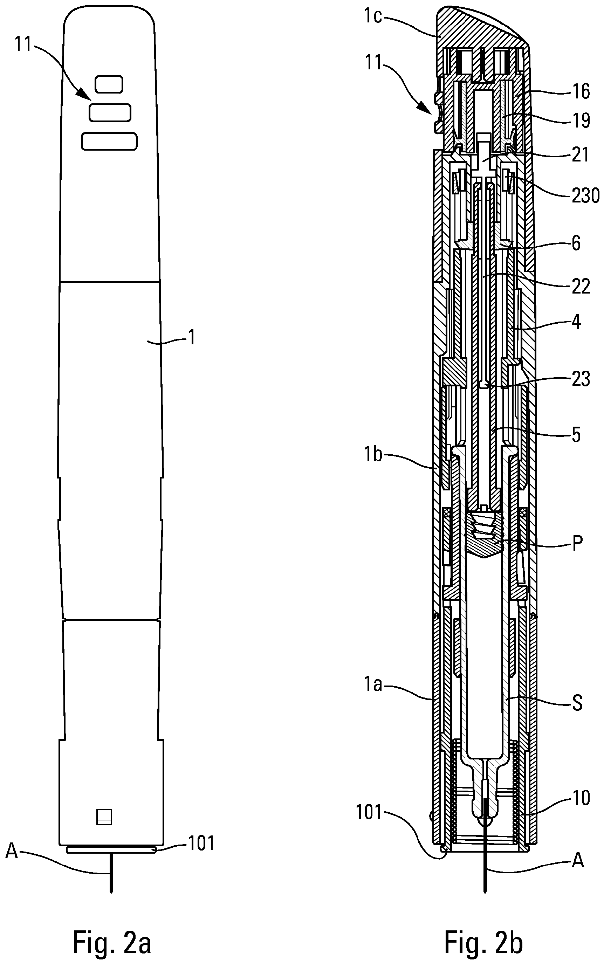

Lorsque l'utilisateur veut utiliser l'autoinjecteur, il prend le dispositif, par exemple au niveau du corps 1 et il appuie le manchon actionneur 10, qui au repos, dans sa première position projetée, fait saillie hors du corps inférieur 1, contre la partie du corps où il veut réaliser l'injection. Sur les figures 1a, 1b et 2a, 2b d'une part et 20a, 20b et 21a, 21b d'autre part, on voit que la pression exercée par l'utilisateur sur le manchon actionneur 10 provoque le coulissement de celui-ci vers l'intérieur du corps 1, ce qui découvre l'aiguille et donc son piquage en raison de la pression exercée par l'utilisateur sur l'autoinjecteur.

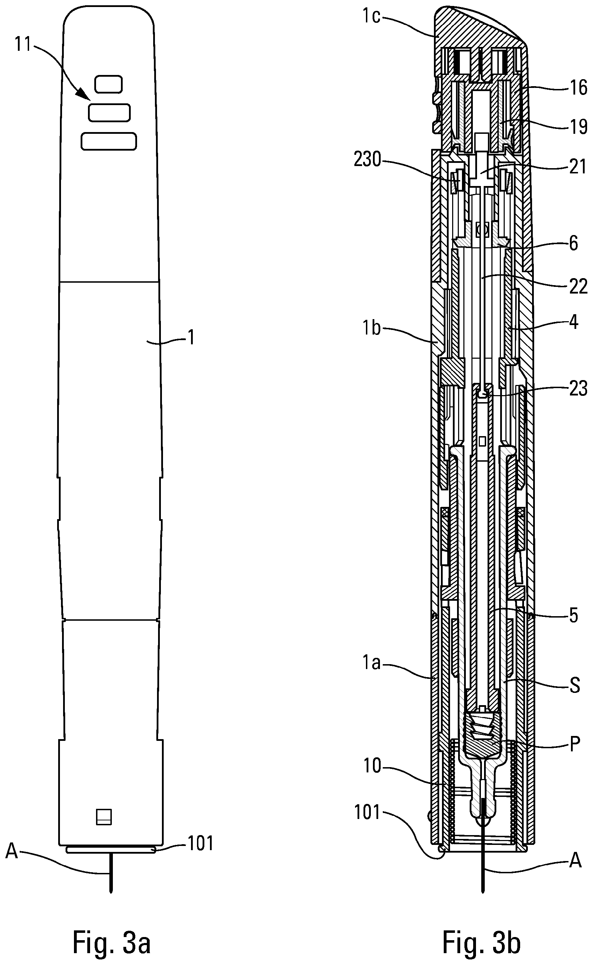

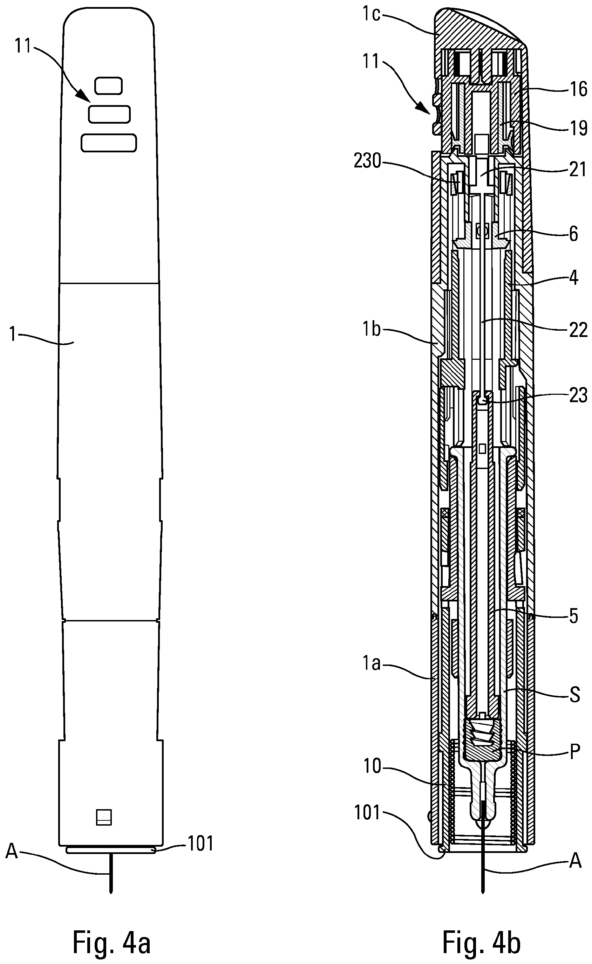

Lorsque le manchon actionneur 10 atteint sa position d'actionnement, qui est sa position d'extrémité à l'intérieur du corps 1, il provoque le déclenchement de la phase d'injection, ce qui est représenté sur les figures 3a, 3b et 4a, 4b d'une part et 22a, 22b et 23a, 23b d'autre part. On constate que la tige de piston 5 coulisse à l'intérieur de la seringue A en poussant le piston P de celle-ci sous l'effet du ressort d'injection 8. Le produit est donc distribué.

A la fin de l'injection, le système retardateur est déclenché, de sorte que le dispositif d'indication ne sera actionné qu'après un temps de retard prédéterminé.

Après indication de la fin d'utilisation, lorsque l'utilisateur retire l'autoinjecteur du site d'injection, le manchon actionneur 10 est à nouveau déplacé hors du corps 1 vers la position de fin d'utilisation, qui est sa seconde position projetée, sous l'effet du ressort du manchon actionneur, avec un verrouillage dudit manchon actionneur 10, ce qui garantit une sécurité absolue pour l'utilisateur et évite tout risque de blessures avec l'aiguille après utilisation du dispositif.

Il est à noter que les première et seconde positions projetées du manchon actionneur, qui, dans l'exemple représenté, sont des positions différentes, pourraient éventuellement être identiques.

La présente invention s'applique à des dispositifs utilisés notamment pour les traitements des maladies auto-immunes, par exemple du type arthrites rhumatoïdes, scléroses multiples, maladie de Crohn, pour les traitements contre le cancer, pour les traitements antiviraux, par exemple du type hépatites, pour les traitements contre le diabète, pour les traitements contre l'anémie ou pour les traitements de crises, par exemple en cas de choc anaphylactique.

Bien que la présente invention ait été décrite en référence à deux modes de réalisation avantageux, il est entendu que ceux-ci ne sont pas limitatifs. En particulier, le manchon actionneur et/ou la serrure d'injection et/ou le dispositif retardateur et/ou le dispositif d'indication sonore et/ou tactile pourrai(en)t être réalisés différemment. Le piquage de l'aiguille et/ou une rétractation de l'aiguille après injection pourrait être commandé par un ou plusieurs bouton(s). D'autres modifications sont également possibles pour l'homme du métier sans sortir du cadre de la présente invention tel que défini par les revendications annexées.

## Revendications

1. Autoinjecteur comportant:
- un corps (1) recevant un réservoir (S), ledit réservoir (S) contenant du produit fluide et comportant un piston (P), tel qu'une seringue pré-remplie,
- une tige de piston (5) adaptée à coopérer avec le piston (P) dudit réservoir (S), ladite tige de piston (5) étant déplaçable par un ressort d'injection (8) entre une position chargée et une position d'injection dans laquelle ladite tige de piston (5) a déplacé le piston (P) du réservoir (S) pour injecter le produit fluide dans un site d'injection,
- un dispositif d'indication visuel, sonore et/ou tactile pour indiquer à l'utilisateur qu'il peut retirer ledit autoinjecteur dudit site d'injection,
ledit autoinjecteur comportant un système retardateur pour retarder l'actionnement dudit dispositif d'indication visuel, sonore et/ou tactile par rapport à la fin de l'injection, ledit système retardateur comportant un réservoir (16), un organe de cisaillement (19) disposé dans ledit réservoir (16) et un fluide disposé dans ledit réservoir (16) autour dudit organe de cisaillement (19), l'un parmi ledit réservoir (16) et ledit organe de cisaillement (19) étant monté rotatif dans ledit corps (1) et l'autre parmi ledit réservoir (16) et ledit organe de cisaillement (19) étant fixe en rotation, la rotation de l'un par rapport à l'autre étant freinée par cisaillement dudit fluide contenu dans ledit réservoir (16), **caractérisé en ce que** ledit réservoir (16) comporte des projections (165) sur sa surface interne et ledit organe de cisaillement (19) comporte des projections (195) sur sa surface externe, lesdites projections (165, 195) générant des restrictions à l'écoulement du fluide, et un cisaillement du fluide lorsqu'une projection (165) du réservoir (16) se trouve face à une projection (195) de l'organe de cisaillement (19).

2. Autoinjecteur selon la revendication 1, dans lequel ledit réservoir (16) est monté rotatif dans ledit corps (1) et ledit organe de cisaillement (19) est fixe en rotation.

3. Autoinjecteur selon l'une quelconque des revendications précédentes, dans lequel ledit système retardateur comprend ledit réservoir (16) contenant ledit fluide, ledit organe de cisaillement (19), un ressort (18; 8), une clé de verrouillage (20) et ladite tige de piston (5).

4. Autoinjecteur selon la revendication 3, dans lequel ladite clé de verrouillage (20) comporte une tête (21), une tige longitudinale (22) et en embout (23) adapté à coopérer avec la tige de piston (5).

5. Autoinjecteur selon la revendication 4, dans lequel, avant déclenchement du système retardateur, la tête (21) de la clé de verrouillage (20) est en position de blocage dans laquelle elle coopère avec un profil correspondant (12) du corps (1) et avec un profil correspondant (161) dudit réservoir (16), de sorte que ledit réservoir (16) est bloqué en rotation par rapport audit corps (1) par ladite clé de verrouillage (20).

6. Autoinjecteur selon la revendication 5, dans lequel, lorsque la tige de piston (5) arrive vers sa position de fin d'injection, elle coopère avec l'embout (23) de la clé de verrouillage (20) pour tirer ladite clé de verrouillage (20) axialement vers le bas hors de sa position de blocage, de sorte que ledit réservoir (16) n'est alors plus bloqué en rotation par ladite clé de verrouillage (20).

7. Autoinjecteur selon l'une quelconque des revendications 3 à 6, dans lequel ledit ressort (18) est un ressort retardateur, réalisé sous la forme d'un ressort à spirale fixé d'une part audit réservoir (16) ou audit organe de cisaillement (19) et d'autre part audit corps (1).

8. Autoinjecteur selon la revendication 7, dans lequel ledit réservoir (16) comporte au moins une patte flexible (166) coopérant avec au moins un profil (106) dudit corps (1), pour fournir une indication sonore du fonctionnement du système retardateur.

9. Autoinjecteur selon la revendication 8, dans lequel ladite au moins une patte flexible (166) coopère avec une pluralité de profils (106) pour générer un bruit continu pendant l'actionnement dudit système retardateur.

10. Autoinjecteur selon la revendication 8, dans lequel ladite patte flexible (166) coopère avec un seul profil (106) pour générer un bruit en fin d'actionnement dudit système retardateur.

11. Autoinjecteur selon l'une quelconque des revendications 3 à 10, dans lequel ledit ressort du système retardateur est ledit ressort d'injection (8), ledit système retardateur comportant en outre un élément indicateur (17) dudit dispositif d'indication visuel, sonore et/ou tactile et un organe support (6) interposé entre ledit élément indicateur (17) et ledit ressort d'injection (8).

12. Autoinjecteur selon la revendication 11, dans lequel ledit élément indicateur (17) est déplaçable axialement dans ledit corps (1), mais n'est pas rotatif, ledit réservoir (16) étant rotatif mais non déplaçable axialement dans ledit corps (1), ledit réservoir (16) comportant sur sa surface externe au moins un filetage externe (163) coopérant avec ledit élément indicateur (17), de telle sorte qu'un déplacement axial dudit élément indicateur (17) autour dudit réservoir (16) entraine une rotation dudit réservoir (16) autour dudit organe de cisaillement (19).

13. Autoinjecteur selon l'une quelconque des revendications 3 à 12, dans lequel ledit élément indicateur (17) comporte des pattes flexibles (179) coopérant, avant actionnement du système retardateur, avec une paroi tronconique ou inclinée (109) du corps (1), la tête (21) de la clé de verrouillage (20), en position de blocage, empêchant la déformation radiale vers l'intérieur desdites pattes flexibles (179).

14. Autoinjecteur selon l'une quelconque des revendications précédentes, dans lequel ledit autoinjecteur comporte un manchon actionneur (10) comportant une extrémité de contact (101) destinée à venir en contact avec le corps de l'utilisateur, ledit manchon actionneur (10) s'étendant au moins partiellement à l'intérieur dudit corps (1) et étant déplaçable par rapport audit corps (1) entre des positions projetées, dans lesquelles ledit manchon actionneur (10) fait au moins partiellement saillie hors dudit corps (1), et une position d'actionnement, dans laquelle ledit manchon actionneur (10) est axialement déplacé vers l'intérieur dudit corps (1), ledit manchon actionneur (10) étant dans une première position projetée avant actionnement de l'autoinjecteur et dans une seconde position projetée après actionnement de l'autoinjecteur.

15. Autoinjecteur selon l'une quelconque des revendications précédentes, dans lequel ledit réservoir (S) comporte une aiguille (A) à travers laquelle ledit produit fluide est injecté dans ledit site d'injection.

## Patentansprüche

1. Automatische Injektionseinrichtung, die Folgendes umfasst:
- einen Körper (1), der einen Vorratsbehälter (S) aufnimmt, wobei der Vorratsbehälter (S) ein Fluidprodukt enthält und einen Kolben (P) aufweist und etwa eine im Voraus befüllte Spritze ist,
- eine Kolbenstange (5), die dafür ausgelegt ist, mit dem Kolben (P) des Vorratsbehälters (S) zusammenzuwirken, wobei die Kolbenstange (5) durch eine Injektionsfeder (8) zwischen einer vorbelasteten Position und einer Injektionsposition, in die die Kolbenstange (5) den Kolben (P) des Vorratsbehälters (S) verlagert hat, um das Fluidprodukt in einen Injektionsort einzuspritzen, verlagerbar ist,
- eine Vorrichtung für visuelle, akustische und/oder taktile Angabe, um dem Benutzer anzugeben, dass er die automatische Injektionseinrichtung von dem Injektionsort zurückziehen kann,
wobei die automatische Injektionseinrichtung ein Verzögerungssystem umfasst, um die Betätigung der Vorrichtung für visuelle, akustische und/oder taktile Angabe in Bezug auf das Ende der Injektion zu verzögern, wobei das Verzögerungssystem einen Vorratsbehälter (16), ein in dem Vorratsbehälter (16) angeordnetes Scherungsorgan (19) und ein in dem Vorratsbehälter (16) um das Scherungsorgan (19) angeordnetes Fluid enthält, wobei entweder der Vorratsbehälter (16) oder das Scherungsorgan (19) in dem Körper (1) drehbar montiert ist und das andere des Vorratsbehälters (16) und des Scherungsorgans (19) drehfest ist, wobei die Drehung des einen in Bezug auf das andere durch eine Scherung des in dem Vorratsbehälter (16) enthaltenen Fluids gebremst wird, **dadurch gekennzeichnet, dass** der Vorratsbehälter (16) auf seiner inneren Oberfläche Vorsprünge (165) aufweist und das Scherungsorgan (19) auf seiner äußeren Oberfläche Vorsprünge (195) aufweist, wobei die Vorsprünge (165, 195) Hindernisse für die Strömung des Fluids und eine Scherung des Fluids, wenn sich ein Vorsprung (165) des Vorratsbehälters (16) gegenüber einem Vorsprung (195) des Scherungsorgans (19) befindet, erzeugen.

2. Automatische Injektionseinrichtung nach Anspruch 1, wobei der Vorratsbehälter (16) in dem Körper (1) drehbar montiert ist und das Scherungsorgan (19) drehfest ist.

3. Automatische Injektionseinrichtung nach einem der vorhergehenden Ansprüche, wobei das Verzögerungssystem den Vorratsbehälter (16), der das Fluid enthält, das Scherungsorgan (19), eine Feder (18; 8), einen Verriegelungsschlüssel (20) und die Kolbenstange (5) umfasst.

4. Automatische Injektionseinrichtung nach Anspruch 3, wobei der Verriegelungsschlüssel (20) einen Kopf (21), eine longitudinale Stange (22) und einen Ansatz (23), der dafür ausgelegt ist, mit der Kolbenstange (5) zusammenzuwirken, umfasst.

5. Automatische Injektionseinrichtung nach Anspruch 4, wobei vor der Auslösung des Verzögerungssystems der Kopf (21) des Verriegelungsschlüssels (20) in einer Blockierposition ist, in der er mit einem dem Körper (1) entsprechenden Profil (12) und mit einem dem Vorratsbehälter (16) entsprechenden Profil (161) zusammenwirkt, derart, dass die Drehung des Vorratsbehälters (16) in Bezug auf den Körper (1) durch den Verriegelungsschlüssel (20) blockiert ist.

6. Automatische Injektionseinrichtung nach Anspruch 5, wobei die Kolbenstange (5) dann, wenn sie ihre Position des Endes der Injektion erreicht, mit dem Ansatz (23) des Verriegelungsschlüssels (20) zusammenwirkt, um den Verriegelungsschlüssel (20) axial nach unten aus seiner Blockierposition zu ziehen, derart, dass die Drehung des Vorratsbehälters (16) durch den Verriegelungsschlüssel (20) nicht mehr blockiert ist.

7. Automatische Injektionseinrichtung nach einem der Ansprüche 3 bis 6, wobei die Feder eine Verzögerungsfeder ist, die in Form einer Spiralfeder verwirklicht ist, die einerseits an dem Vorratsbehälter (16) oder an dem Scherungsorgan (19) und andererseits an dem Körper (1) befestigt ist.

8. Automatische Injektionseinrichtung nach Anspruch 7, wobei der Vorratsbehälter (16) wenigstens eine biegsame Lasche (166) aufweist, die mit wenigstens einem Profil (106) des Körpers (1) zusammenwirkt, um eine akustische Angabe des Betriebs des Verzögerungssystems bereitzustellen.

9. Automatische Injektionseinrichtung nach Anspruch 8, wobei die wenigstens eine biegsame Lasche (166) mit mehreren Profilen (106) zusammenwirkt, um während der Betätigung des Verzögerungssystems ein ununterbrochenes Geräusch zu erzeugen.

10. Automatische Injektionseinrichtung nach Anspruch 8, wobei die biegsame Lasche (166) mit einem einzigen Profil (106) zusammenwirkt, um am Ende der Betätigung des Verzögerungssystems ein Geräusch zu erzeugen.

11. Automatische Injektionseinrichtung nach einem der Ansprüche 3 bis 10, wobei die Feder des Verzögerungssystems die Injektionsfeder (8) ist, wobei das Verzögerungssystem außerdem ein Angabeelement (17) der Vorrichtung für visuelle, akustische und/oder taktile Angabe sowie ein Trägerorgan (6), das zwischen das Angabeelement (17) und die Injektionsfeder (8) eingefügt ist, umfasst.

12. Automatische Injektionseinrichtung nach Anspruch 11, wobei das Angabeelement (17) in dem Körper (1) axial, jedoch nicht rotatorisch verlagerbar ist, während der Vorratsbehälter (16) in dem Körper (1) rotatorisch, jedoch nicht axial verlagerbar ist, wobei der Vorratsbehälter (16) auf seiner äußeren Oberfläche wenigstens ein Außengewinde (163) aufweist, das mit dem Angabeelement (17) zusammenwirkt, derart, dass eine axiale Verlagerung des Angabeelements (17) um den Vorratsbehälter (16) eine Drehung des Vorratsbehälters (16) um das Scherungsorgan (19) zur Folge hat.

13. Automatische Injektionseinrichtung nach einem der Ansprüche 3 bis 12, wobei das Angabeelement (17) biegsame Laschen (179) aufweist, die vor der Betätigung des Verzögerungssystems mit einer kegelstumpfförmigen oder geneigten Wand (109) des Körpers (1) zusammenwirken, wobei der Kopf (21) des Verriegelungsschlüssels (20) in der Blockierposition die radiale Verformung nach innen der biegsame Laschen (179) verhindert.

14. Automatische Injektionseinrichtung nach einem der vorhergehenden Ansprüche, wobei die automatische Injektionseinrichtung eine Betätigungshülse (10) umfasst, die ein Kontaktende (101) aufweist, das dazu bestimmt ist, mit dem Körper des Benutzers in Kontakt zu gelangen, wobei sich die Betätigungshülse (10) wenigstens teilweise innerhalb des Körpers (1) erstreckt und in Bezug auf den Körper (1) zwischen vorstehenden Positionen, in denen die Betätigungshülse (10) wenigstens teilweise von dem Körper (1) vorsteht, und einer Betätigungsposition, in der die Betätigungshülse (10) axial in den Innenraum des Körpers (1) verlagert ist, verlagerbar ist, wobei die Betätigungshülse (10) vor der Betätigung der automatischen Injektionseinrichtung in einer ersten vorstehenden Position ist und nach der Betätigung der automatischen Injektionseinrichtung in einer zweiten vorstehenden Position ist.

15. Automatische Injektionseinrichtung nach einem der vorhergehenden Ansprüche, wobei der Vorratsbehälter (S) eine Nadel (A) enthält, durch die das Fluidprodukt in den Injektionsort eingespritzt wird.

## Claims

1. An autoinjector comprising:
• a body (1) receiving a reservoir (S), said reservoir (S) containing fluid and including a piston (P), such as a pre-filled syringe,
• a piston rod (5) that is adapted to co-operate with the piston (P) of said reservoir (S), said piston rod (5) being movable by an injection spring (8) between a primed position and an injection position in which said piston rod (5) has moved the piston (P) of the reservoir (S) so as to inject the fluid into an injection site,
• a visual, audible and/or tactile indicator device for indicating to the user that said autoinjector may be removed from said injection site,
said autoinjector including a retarding system so as to delay actuating said visual, audible and/or indicator device relative to the end of injection, said retarding system comprising a dashpot (16), a shear member (19) arranged in said dashpot (16) and a fluid arranged in said dashpot (16) around said shear member (19), one of said dashpot (16) and of said shear member (19) being rotatably mounted in said body (1) and the other one of said dashpot (16) and of said shear member (19) being stationary in rotation, the turning of one relative to the other being braked by shearing said fluid contained in said dashpot (16), **characterized in that** said dashpot (16) includes projections (165) on its inside surface and said shear member (19) includes projections (195) on its outer surface, said projections (165, 195) generating impediments to the flow of the fluid, and shearing the fluid when a projection (165) of the dashpot (16) faces a projection (195) of the shear member (19).

2. An autoinjector according to claim 1, wherein said dashpot (16) is rotatably mounted in said body (1), and said shear member (19) is stationary in rotation.

3. An autoinjector according to any preceding claim, wherein said retarding system comprises said dashpot (16) containing said fluid, said shear member (19), a spring (18; 8), a locking key (20), and said piston rod (5).

4. An autoinjector according to claim 3, wherein said locking key (20) comprises a head (21), a longitudinal rod (22), and an endpiece (23) that is adapted to co-operate with the piston rod (5).

5. An autoinjector according to claim 4, wherein, prior to triggering the retarding system, the head (21) of the locking key (20) is in its blocking position in which it co-operates with a corresponding profile (12) of the body (1) and with a corresponding profile (161) of said dashpot (16), such that said dashpot (16) is prevented from turning relative to said body (1) by said locking key (20).

6. An autoinjector according to claim 5, wherein, when the piston rod (5) arrives towards its end-of-injection position, it co-operates with the endpiece (23) of the locking key (20) so as to pull said locking key (20) axially downwards out from its blocking position, such that said dashpot (16) is thus no longer prevented from turning by said locking key (20).

7. An autoinjector according to any one of claims 3 to 6, wherein said spring (18) is a retarding spring, made in the form of a spiral spring that is fastened firstly to said dashpot (16) or to said shear member (19) and secondly to said body (1).

8. An autoinjector according to claim 7, wherein said dashpot (16) includes at least one flexible tab (166) that co-operates with at least one profile (106) of said body (1) so as to provide an audible indication that the retarding system is operating.

9. An autoinjector according to claim 8, wherein said at least one flexible tab (166) co-operates with a plurality of profiles (106) so as to generate a continuous noise while said retarding system is being actuated.

10. An autoinjector according to claim 8, wherein said flexible tab (166) co-operates with a single profile (106) so as to generate a noise at the end of actuating said retarding system.

11. An autoinjector according to any one of claims 3 to 10, wherein said spring of the retarding system is said injection spring (8), said retarding system further comprising an indicator element (17) of said visual, audible, and/or tactile indicator device, and a support member (6) that is interposed between said indicator element (17) and said injection spring (8).

12. An autoinjector according to claim 11, wherein said indicator element (17) is axially movable in said body (1), but it is not rotary, said dashpot (16) being rotary but not axially movable in said body (1), the outside surface of said dashpot (16) including at least one external thread (163) that co-operates with said indicator element (17), such that an axial movement of said indicator element (17) around said dashpot (16) causes said dashpot (16) to turn around said shear member (19).

13. An autoinjector according to any one of claims 3 to 12, wherein said indicator element (17) includes flexible tabs (179) that, before actuation of the retarding system, co-operate with a frustoconical or sloping wall (109) of the body (1), the head (21) of the locking key (20), in its blocking position, preventing said flexible tabs (179) from deforming radially inwards.

14. An autoinjector according to any preceding claim, wherein said autoinjector includes an actuator sleeve (10) that includes a contact end (101) for coming into contact with the user's body, said actuator sleeve (10) extending inside said body (1) at least in part and being movable relative to said body (1) between projecting positions, in which said actuator sleeve (10) projects out from said body (1) at least in part, and an actuated position in which said actuator sleeve (10) is moved axially into said body (1), said actuator sleeve (10) being in a first projecting position before actuation of the autoinjector and in a second projecting position after actuation of the autoinjector.

15. An autoinjector according to any preceding claim, wherein said reservoir (S) includes a needle (A) through which said fluid is injected into said injection site.
